# EUROPEAN PATENT APPLICATION

(11) **EP 4 585 224 A1**
(43) Date of publication of application: **16.07.2025**
(21) Application number: 23863163.4
(22) Date of filing: 05.09.2023
(51) Int. Cl.: A61K 45/00, A61P 23/02, A61P 43/00, A61P 17/00, A61P 17/04, A61K 9/70, A61M 37/00, A61P 25/04, A61P 29/00, A61P 29/02, A61K 31/135, A61K 31/167, A61K 31/245, A61K 31/4402, A61K 47/32, A61K 47/34

(54) **LOCAL APPLICATION SYSTEM**

(30) Priority: 08.09.2022 JP 2022143339; 11.11.2022 JP 2022180765
(71) Applicant: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); HASEGAWA, Junya, Kyoto-shi, Kyoto 601-8014 (JP); TANAKA, Hiroshi, Kyoto-shi, Kyoto 601-8014 (JP); LI, Ying-zhe, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2023/032308
(87) International publication number: WO 2024/053625

(57) **Abstract**

Provided is a means for shortening the time of the development of the action of a local anesthetic agent, an antihistamine drug or the like against skin and oral mucosa. Provided are: a minute projection patch which is intended to be applied to skin or oral mucosa and includes a substrate and minute projections stereoscopically erected on the substrate, in which each of the substrate and the minute projections is made of a water-insoluble thermoplastic polymer substance having an elastic modulus of 1.5 × 10⁴ kgf/cm² or less; and a local application system for shortening the time of the action of a drug against the skin or oral mucosa, which contains the minute projection patch and a liniment (a gel, an ointment or the like containing a drug) .

## Description

### TECHNICAL FIELD

The present invention relates to a patch including minute projections having no hardness for puncturing skin. Furthermore, the present invention relates to a local application system using the patch.

### BACKGROUND ART

As a local anesthetic preparation used for pain relief during laser therapy, a currently commercially available preparation is used, but it takes 40 to 60 minutes to exert an anesthetic effect after the application of the preparation to a skin surface. Even when a local anesthetic for patch (tape agent containing 60% of lidocaine, applied for pain relief at the time of intravenous indwelling needle puncture) is used for the purpose of pain relief, it takes 60 minutes in the present situation as well. For the treatment of insect bites, dermatitis, itch, and the like, an external preparation for skin is used, which uses a local anesthetic agent, an antihistamine drug, or the like as a drug and is applied to an affected area. The same time is required for developing the effect on these.

Dental local anesthetics are also widely used. As the drug, a (gel) ointment using lidocaine or ethyl aminobenzoate is generally used, but the time of the development of the effect is 10 minutes, which is shorter than the development of the effect on the skin. However, the development of action in a shorter time is required from both doctors and patients.

The present inventors have developed a microneedle array containing a local anesthetic agent for intraoral application, and have succeeded in achieving an anesthetic effect in a short time (Patent Document 1). In Patent Document 1, a microneedle made of a water-soluble resin contains the local anesthetic agent.

### PRIOR ART DOCUMENT

### PATENT DOCUMENT

Patent Document 1: Japanese Patent Application Laid-Open No. 2019-084352

### SUMMARY OF THE INVENTION

### PROBLEM TO BE SOLVED BY THE INVENTION

In Patent Document 1, from the standpoint of a doctor, a microneedle local anesthetic as a pharmaceutical product already manufactured is administered to a patient. For the purpose of administering a local administration formulation containing various (gel) ointments and liquid preparations to develop an effect in a shorter time, there is a need for a new administration method other than that of Patent Document 1, which is appropriately prepared and applied in medical practice using a pharmaceutical product such as an existing ointment.

The present invention has been made to solve the problem related to the local application preparation, and an object thereof is to provide a means for shortening the time to develop the effect of a preparation such as an ointment, a cream, a liquid preparation, a gel, or a spray for local administration for the purpose of local anesthesia, antihistamine, anti-inflammation, antibacterial, pain relief, antipruritus, or the like.

### MEANS FOR SOLVING THE PROBLEM

The present invention is characterized in that in order to shorten the time of the development of the action of a local anesthetic, an antihistamine, an anti-inflammatory agent, an antibacterial agent, a pain relieving agent, an antipruritic agent, and the like on skin and oral mucosa, an ointment (gel) or a liquid preparation containing these drugs is overlaid on an application site with a minute projection patch when applying the ointment or the liquid preparation to an affected area.

As the patch including minute projections, a microneedle patch is known. A material constituting the microneedle is made of silica, metal, plastic, a biosoluble substance, or the like, and the conventional microneedle is characterized by having hardness capable of skin puncture. In the present invention, the minute projections have hardness that cannot be punctured even when applied to skin or oral mucosa. An object of the present invention is to improve the transdermal and mucosal absorption of a drug to be simultaneously administered by locally stretching and thinning the skin or oral mucosa by pressing the skin with the minute projections although the skin cannot be punctured. The advantage of using a substance inferior in hardness is that the transdermal transmucosal absorption of a drug can be promoted without damaging the skin.

The present invention is as follows.
[1] A minute projection patch intended to be applied to skin or oral mucosa, including a substrate and minute projections stereoscopically erected on the substrate, in which the substrate and the minute projections are made of a water-insoluble thermoplastic polymer substance having an elastic modulus of 1.5 × 10⁴ kgf/cm² or less.
[2] The minute projection patch according to [1], in which the minute projections have a tip portion diameter of 10 µm to 300 µm, the minute projections have a length of 50 µm to 1500 µm, and the substrate has a thickness of 20 to 500 µm.
[3] The minute projection patch according to [1] or [2], in which the water-insoluble thermoplastic polymer substance is selected from the group consisting of polyolefin, soft silicon, and polyurethane.
[4] The minute projection patch according to any one of [1] to [3], in which the minute projection patch is lined with an adhesive tape.
[5] The minute projection patch according to [4], in which an adhesive constituting the adhesive tape contains a hydrophilic substance.
[6] The minute projection patch according to [5], in which the hydrophilic substance is at least one selected from the group consisting of polyacrylic acid, hyaluronic acid and a sodium salt thereof, carboxymethyl cellulose and a sodium salt thereof, polyvinyl alcohol, polyvinyl pyrrolidone, dextrin, sodium chondroitin sulfate, collagen, and crosslinked products thereof.
[7] The minute projection patch according to [4], in which an adhesive base material constituting the adhesive tape is a foam.
[8] The minute projection patch according to [4], in which an adhesive base material constituting the adhesive tape is a nonwoven fabric.
[9] A local application system for shortening the time of action of a drug against skin or oral mucosa, containing the minute projection patch according to any one of [1] to [8] and a liniment.
[10] The local application system according to [9], in which the drug is a local anesthetic agent or an antihistamine drug and is intended for pain relief or short-term achievement of anti-itch.
[11] The local application system according to [10], in which the local anesthetic agent is lidocaine or ethyl aminobenzoate.
[12] The local application system according to [10], in which the antihistamine drug is selected from the group consisting of diphenhydramine and a salt thereof, and chlorpheniramine and a salt thereof.

### EFFECT OF THE INVENTION

The local application system of the present invention makes it possible to shorten the time of the development of the action of a liniment such as a local anesthetic, an antihistamine, an anti-inflammatory agent, an antibacterial agent, a pain relieving agent, or an antipruritic agent on skin or oral mucosa.

### BRIEF DESCRIPTION OF THE DRAWINGS

FIG. 1 is a view of a minute projection patch according to an embodiment of the present invention. In FIGS. 1A and 1B, an adhesive tape is adhered to the entire back surface of a substrate of the minute projection patch. In FIG. 1C, an adhesive tape is adhered to a part of the back surface of the substrate of the minute projection patch. In FIG. 1D, an adhesive foam is adhered to the back surface of the substrate of the minute projection patch.
FIG. 2 is a micrograph of the minute projection patch of the present invention.

### MODE FOR CARRYING OUT THE INVENTION

A polymer substance having hardness that cannot be punctured even when applied to skin or oral mucosa, which is a characteristic of the present invention, and being water-insoluble was examined. Being water-insoluble is a necessary condition for a material of the present invention because minute projections do not dissolve or swell even when they come into contact with the skin or oral mucosa for several minutes to several tens of minutes. When water-dissolvable minute projections are applied to the skin or mucosa, they are likely to swell and dissolve due to moisture on the surface, and a drug diffuses to the skin or mucosa side. At the same time, a phenomenon of back diffusion to the minute projection portions may also occur. Even when a minute projection patch is produced using a water-soluble material such as a water-soluble resin such as hyaluronic acid or carboxymethyl cellulose, the minute projections are brought into contact with a gel ointment, or brought into contact with the skin or oral mucosa, so that the minute projections dissolve due to the moisture to lose their shapes, and do not play a role of promoting permeation.

As the quantitative measure of the hardness, various polymer substances having different elastic moduli (unit: kgf/cm²) were formed into minute projection patches by press molding, and the patches were pressed against the skin to evaluate punctuability. As a result, it was found to be appropriate to use a water-insoluble thermoplastic polymer substance having an elastic modulus of 1.5 × 10⁴ kgf/cm² or less. Specifically, a minute projection patch made of polyolefin, soft silicon, polyurethane, or the like is suitable. The elastic moduli of various plastics measured by the inventors or confirmed from a product catalog were as follows (unit: 10⁴ kgf/cm²). In the polyolefin, the elastic moduli of polypropylene, high-density polyethylene, and ethylene-vinyl acetate copolymer are respectively 1.2, 1.0, and 0.05. The elastic moduli of a soft silicon resin and polyurethane resin are respectively 0.06 and 0.08. These resins can be suitably used in the present invention.

Polyvinyl chloride (3.0), polyacetal (3.3), polyglycolic acid (7.6), and the like are hard and minute projections manufactured from these materials are not preferred in the present invention since they puncture the skin and thus may cause bleed on application.

In a minute projection patch composed of the water-insoluble thermoplastic polymer substance having an elastic modulus of 1.5 × 10⁴ kgf/cm² or less, the outermost layer of the skin or oral mucosa (stratum corneum in the skin) is stretched by the compression of the skin or oral mucosa by the minute projections, whereby the thickness of the outermost layer can be reduced and the intradermal penetration of a drug can be enhanced. In the case of needle-like minute projections, the tip portion of the minute projections enters a shallow portion of the mucosa to form micropores, which makes it possible to promote the penetration of the drug. As is well known, the largest barrier of drug permeation to the skin is the stratum corneum, and if its thickness can be 1/2, theoretically the drug permeation increases by a factor of 2.

By using the liniment in combination with the minute projection patch, the minute projection patch covers the drug to prevent the drug from flowing to the periphery, and thus the drug can be more safely administered. In particular, in the case of dental surface anesthesia, a local anesthetic agent easily flows to other sites such as the throat only by application, and the effect of the drug at the target site can be intensively exerted by being fixed by the minute projection patch.

Examples of the dosage form of the liniment include an ointment, a cream, a liquid preparation, a gel, and a spray.

The present invention provides a local application system for shortening the time of the action of a drug against skin or oral mucosa, containing the minute projection patch of the present invention and the liniment (an ointment, a cream, a liquid preparation, a gel, or a spray containing a drug). The local application system is a formulation system that is administered to the skin or oral mucosa, and relates to reduction in a time for developing the effect of a preparation such as an ointment, a cream, a liquid preparation, a gel, or a spray for local administration for the purpose of local anesthesia, antihistamine, anti-inflammation, antibacterial, pain relief, antipruritus, or the like.

The minute projection patch includes a substrate and minute projections stereoscopically erected on the substrate.

The thickness of the substrate is preferably 20 µm to 500 µm, and more preferably 50 µm to 300 µm. When the thickness of the substrate is 20 µm or more, the substrate is easy to handle, and when the thickness is 500 µm or less, the substrate is easily brought into close contact with an oral mucosa portion having large unevenness.

The length of the minute projections is preferably 50 µm to 1500 µm, and more preferably 100 µm to 1000 µm. The length of the minute projections of 50 µm or more is sufficient as a projection height for obtaining a surface layer skin thinning effect. When the length of the minute projection is 1500 µm or less, the curvature of the minute projection can be prevented.

The tip portion of the minute projection should not be sharp so as not to deeply enter the skin or oral mucosa when the skin is pressed. Therefore, the shape of the minute projection is preferably a cylindrical shape, a conical shape, or a truncated cone shape. In the case of a projection having such a shape, the diameter of the tip portion is preferably 10 µm or more and 300 µm or less, and more preferably 30 µm to 200 µm.

As long as the projections are minute projections made of a water-insoluble thermoplastic polymer substance having an elastic modulus of 1.5 × 10⁴ kgf/cm² or less and have a tip portion diameter of 10 µm or more, there is no possibility that the projections are deeply inserted into the skin. When the tip portion diameter is 300 µm or less, sufficient unevenness can be formed in the skin.

The density of the minute projections is 20 to 1000 projections/cm². When the density is 20 projections/cm² or more, the effect of the present invention can be exerted. When the density is 1000 projections/cm² or less, sufficient unevenness of the skin is likely to appear.

The minute projections are suitably manufactured by microneedle manufacturing by thermocompression-bonding a thermoplastic plastic to a concave mold, but as another production method, the minute projections may be manufactured by a thermocompression -bonding method of a thermoplastic plastic to a metal surface whose surface is roughened by sandblasting or the like.

The area of the minute projection patch corresponds to the area of the substrate, and is not particularly limited, but is desirably 1 to 5 cm² in intraoral application.

In the minute projection patch of such an aspect, the applied drug can be reliably held at the site, the drug administration efficiency can be remarkably improved, and in the case of the local anesthetic agent, the time of the development of the anesthetic effect can be shortened. In the case of pain relief and antipruritic agents, pain relief, reduction of anti-itch time, and the like can be realized. In order to apply the minute projection patch to the skin, the patch may be lined with an adhesive tape at the periphery to fix the patch to the skin. The adhesive tape is not essential, but fixing with the adhesive tape is preferable for stably fixing the patch to the skin.

The adhesive tape contains an adhesive and a base material, and is obtained by applying the adhesive onto the base material. As the adhesive, rubber-based, silicon-based, acryl-based, urethane-based adhesives, and the like can be used, but a hydrophilic adhesive is preferable when the adhesive is applied to oral mucosa. Examples of the hydrophilic adhesive include a hydrophilic acrylic adhesive, HiPAS10 (trade name) adhesive (acrylic adhesive containing methyl methacrylate as a main monomer, CosMED Pharmaceutical Co., Ltd.), and HiPAS-PU (trade name) (urethane-based adhesive, CosMED Pharmaceutical Co., Ltd.).

The adhesive is directly applied onto the base material and used, and particularly when the adhesive is applied to the oral cavity, the oral cavity is extremely hydrophilic and contains a large amount of water on the surface, so that the water may cover the surface of the adhesive to lose the adhesion. In order to prevent this and enable stable skin application for a long time, it is effective to blend and mix a hydrophilic substance in the adhesive in advance and apply the blended product to the base material. The hydrophilic substance in the present invention refers to a substance that dissolves or swells when added to water. As the hydrophilic substance, low-molecular hydrophilic substances such as glucose and glycerin; hydrophilic polymer substances such as polyacrylic acid, hyaluronic acid (sodium salt), carboxymethyl cellulose (sodium salt), polyvinyl alcohol, polyvinyl pyrrolidone, dextrin, sodium chondroitin sulfate, and collagen; or a crosslinked product of a hydrophilic polymer substance or the like is used. In particular, a hydrophilic polymer substance is more preferable from the viewpoint of being added to a nonaqueous solution such as an acrylic adhesive. The role of the hydrophilic substance is to absorb moisture in the oral cavity to maintain adhesion as the adhesive for a long time. The addition amount of the hydrophilic substance is preferably 3 to 80% by mass, and more preferably 5 to 70% by mass. When the addition amount of the hydrophilic substance is small, the above effect is hardly exerted, and when the addition amount is too large, the adhesion as the adhesive is lost. At the same time, the hydrophilic substance tends to remain on the skin.

A plasticizer may be added to the adhesive. As the plasticizer, a commonly used plasticizer, for example, isopropyl myristate, isopropyl palmitate, octyl myristate, octyldodecyl lactate, or the like can be used.

The base material to which the adhesive is applied is desirably a flexible film, a foam, or nonwoven fabric from the viewpoint of application to a bent portion such as the skin or the oral cavity. In order to stabilize the adhesion of the adhesive tape in the oral cavity by the absorption of moisture in the oral cavity, the use of a foam or a nonwoven fabric is more desirable, and a material having water absorbency is more preferable. The foam can exert the effect of preventing the flow of the preparation when an application drug such as a cream or a liquid formulation is used as an overlay. Since the foam is thicker than the film, it is required that the hardness of the film is not too large so as not to cause a sense of discomfort in the oral cavity. When the elastic modulus of the foam is 20 kgf/cm² or less, the sense of discomfort in the oral cavity does not occur. The elastic modulus can be measured from a stress-variation curve when a foam is punched into a dumbbell and pulled with a tensile tester. The foam may be either an open cell foam or a closed cell foam. The side in contact with the oral cavity may be porous. The thickness is preferably 300 µm or less and 5 µm or more (may be 100 µm or less and 5 µm or more) in the case of the film, and is preferably 10 mm or less and 0.1 mm or more in the case of the foam. In the case of the nonwoven fabric, the thickness is preferably 20 mm or less and 0.05 mm or more. More preferably, the thickness is 100 µm or less and 10 µm or more in the case of the film, and 6 mm or less and 0.5 mm or more in the case of the foam. In the case of the nonwoven fabric, the thickness is preferably 10 mm or less and 0.08 mm or more. The size of the adhesive/base material is desirably 1 to 10 mm from the edge of the minute projection patch in order to fix the minute projection patch.

In actual application, a liniment (gel, ointment, or the like) is placed on a minute projection surface, spread to form a planar shape, then applied to the skin, and pressed from the back surface of the substrate to bring the minute projection patch into close contact with the skin. The shape of the minute projection patch may be a square, a rectangle, a circle, an ellipse, a cylinder, a sphere, or the like. When the minute projection patch is lined with the adhesive tape and brought into close contact with the skin, the adhesion is further improved. As a result, the skin is elongated to promote the permeation of the drug into the skin. Even if the skin is pressed with the minute projections, the skin is not broken by the minute projections and bleeding does not occur as long as the density of the minute projections is high and the length of the minute projections is 1500 µm or less.

The minute projection patch for oral mucosa application is preferably a rectangular patch having a minor axis of 15 mm or less and a major axis of 30 mm or less in order to closely fit the tooth floor. The thickness of the substrate is preferably 500 µm or less, and particularly preferably 300 µm or less. Furthermore, the substrate also requires flexibility, and the water-insoluble thermoplastic polymer substance constituting the substrate also desirably has an elastic modulus of 1.5 × 10⁴ Kgf/cm² or less. The minute projection forming polymer substance and the substrate forming polymer substance are desirably the same, but different water-insoluble thermoplastic polymer substances may be used as long as the above conditions are satisfied.

Examples of the method for molding the minute projections in the present invention include press molding using a mold, mold molding in which a solution obtained by dissolving a polymer substance in a solvent is filled in a mold and dried, and injection molding. When a soft silicon resin or a urethane resin is used as a raw material, a raw material before curing is placed in a mold and cured to form a minute projection patch. A thermoplastic resin is placed in a mold, filled upon heating, cooled, and then taken out.

Press molding is the easiest. In particular, press molding using a mold is a more appropriate manufacturing method in order to thin the substrate of the minute projections to make the substrate flexible.

The minute projections are suitably manufactured by microneedle manufacturing in which a thermoplastic plastic is thermocompression-bonded to a concave mold, but as other production methods, the minute projections may be produced by sandblasting, chemical treatment, a method of thermocompression-bonding a thermoplastic plastic to a metal surface roughened with a laser or the like, or the like. The maximum height (Rz) of the unevenness generated in the roughening by these methods is defined as a minute projection length in the present invention.

Examples of the local anesthetic agent as the drug that can be used in the present invention include lidocaine, mepivacaine, dibucaine, bupivacaine, ropivacaine, procaine, chloroprocaine, tetracaine, and benzocaine (ethyl aminobenzoate) .

The antihistamine drug is preferably diphenhydramine and a salt thereof, chlorpheniramine and a salt thereof, isothipentyl and a salt thereof, diphenylpyraline and a salt thereof, and diphenylimidazole and a salt thereof.

As anti-inflammation, pain relief, and antipruritic agents, adrenocortical hormone agents such as betamethasone ester, nonsteroidal agents such as diclofenac and indometacin, external vitamin agents such as vitamin A, and antibacterial agents such as an antibiotic are preferable.

As a method of use, after the liniment is applied to the skin or oral mucosa, a minute projection patch may be placed so as to cover the skin or oral mucosa and pressed from the back surface of the substrate. Alternatively, the liniment may be directly applied to the minute projection surface, applied to the skin or oral mucosa, and lined with an intraoral adhesive tape.

### EXAMPLES

Hereinafter, the present invention will be described in more detail by the following Examples. These Examples are merely examples for specifically describing the present invention, and the scope of the present invention is not limited to these Examples.

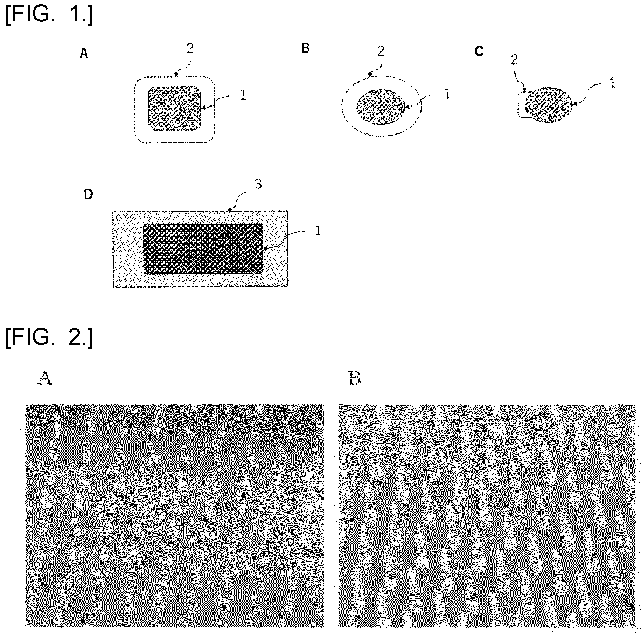

In a minute projection patch manufactured in the present Examples, a plurality of minute projections are formed on a sheet-shaped substrate, and an adhesive tape is laminated on a surface of the sheet-shaped substrate opposite to a surface on which the minute projections are formed (see FIGS. 1A to 1C). Hereinafter, an example of an embodiment in which the minute projection patch of the present invention is used for local anesthesia application will be described.

### Example 1

A first embodiment of a minute projection patch of the present invention will be described with reference to the drawings.

In the minute projection patch illustrated in FIG. 1B, a plurality of minute projections having a length of 700 µm are stereoscopically erected on a circular substrate, each projection has a conical shape, a diameter of a projection tip is 35 µm, and a projection interval is 500 µm. A micrograph of the minute projections is shown in FIG. 2B.

A polypropylene sheet having a thickness of 100 µm was placed on a metal concave mold, heated to 180°C, and press-molded to mold a sheet in which the minute projections are stereoscopically erected. The sheet was cut to a diameter of 10 mm to manufacture minute projection patches. The length of the projection was 700 µm, and the thickness of the substrate was 90 µm. The minute projection patch was used by bonding a commercially available adhesive plaster onto a tape cut into a diameter of 16 mm.

In order to actually use the minute projection patch, 0.1 g of an external local anesthetic

"EMLA CREAM" (containing 25 mg of lidocaine and 25 mg of propitocaine in 1 g of ointment) was spread over the entire surface of the minute projections. In this state, the minute projections on which the drug was placed were applied to the upper arms of three volunteers, and the substrate (the back surface of the minute projections) was pressed to bring the ointment and the minute projection patch into close contact with the skin. After the skin application, the tape was partially peeled off every 5 minutes, the drug application site was lightly pressed with a toothpick, and the presence or absence of pain was self-evaluated. The times for which the pain was not felt were 25 minutes, 25 minutes, and 30 minutes in the three volunteers. For comparison, at the same time, 0.1 g of EMLA CREAM was applied to the round part with a diameter of 1 cm on the upper arm skin, and the time for which the pain was not felt was evaluated every 5 minutes. The times for which the pain was not felt were 45 minutes, 50 minutes, and 50 minutes in the three volunteers.

### Example 2

A second embodiment of a minute projection patch of the present invention will be described with reference to the drawings.

In the minute projection patch illustrated in FIG. 1A, a plurality of minute projections having a length of 300 µm are stereoscopically erected on a rectangular substrate, each projection has a truncated conical shape, a diameter of a projection tip is 25 µm, and a projection interval is 500 µm. A micrograph of the minute projections is shown in FIG. 2A. A high density polyethylene sheet having a thickness of 60 µm was placed on a metal concave mold, heated to 150°C, and press-molded to mold a sheet in which the minute projections are stereoscopically erected. The sheet was cut into 10 mm × 20 mm to manufacture a minute projection patch having a minute projection length of 300 µm and a substrate thickness of 50 µm. The minute projection patch was used by bonding a commercially available adhesive plaster onto a tape cut into 16 × 26 mm.

In order to actually use the minute projection patch, 0.1 g of a dental local anesthetic "HURRICAINE GEL" (containing 200 mg of ethyl aminobenzoate in 1 g of gel ointment) was spread over the entire surface of the minute projections. In this state, the minute projections on which the drug was placed were applied to the upper jaw parts of three volunteers, and the substrate (the back surface of the minute projections) was pressed to bring the ointment and the minute projection patch into close contact with the skin. After the skin application, the tape was partially peeled off every 5 minutes, the drug application site was lightly pressed with a toothpick, and the presence or absence of pain was self-evaluated. The times for which the pain was not felt were 5 minutes in all the three volunteers. For comparison, at the same time, 0.1 g of HURRICAINE GEL was applied to the round part with a diameter of 1 cm on the upper arm skin, and the time for which the pain was not felt was evaluated every 5 minutes. The times for which the pain was not felt were 5 minutes, 10 minutes, and 10 minutes in the three volunteers.

### Comparative Example 1

Using hyaluronic acid as a material, a sheet in which minute projections having a length of 300 µm were stereoscopically erected on a rectangular substrate was molded by mold molding. The shape of the projection was the same as that in Example 2. The sheet was manufactured by cutting into 10 mm × 20 mm, and a commercially available adhesive plaster was adhered onto a "tape" cut into 16 × 26 mm to be used as a minute projection patch. On the patch, 0.1 g of a dental local anesthetic "HURRICAINE GEL" (containing 200 mg of ethyl aminobenzoate in 1 g of gel ointment) was spread over the entire "minute projection" portions.

When the minute projections were observed after 5 minutes, hyaluronic acid was swollen and dissolved by moisture in the gel, and the shape of the minute projections completely disappeared.

### Examples 3 to 9

A minute projection patch of 10 mm × 20 mm similar to that manufactured in Example 2 was bonded onto an adhesive tape cut into 16 × 26 mm for use. Over the entire minute projections, 0.1 g of "HURRICAINE GEL" (trade name, containing 200 mg of ethyl aminobenzoate in 1 g of gel ointment) was spread. Seven types of adhesive tapes were used. The composition of the adhesive of the adhesive tape used and the type of the base material are shown in Table 1.

The minute projection patch with an adhesive tape of each of Examples 3 to 9 was moved left and right together with the adhesive tape after 1 minute in a state of being pressed against the upper jaw, and the strength of adhesion of the tape to the upper jaw was evaluated. The results were classified as follows.
(1) The tape was slid and moved on the upper jaw portion by pressing with a light force.
(2) Pressing with a light force did not cause the tape to be moved, but strong pressing caused the tape to be slid and moved on the upper jaw portion.
(3) The adhesion of the tape to the upper jaw portion was strong, and thus the tape was not moved even by strong pressing.
(4) When the tape was pressed with a strong force, a part of polyacrylic acid adhered and remained on the upper jaw portion.

**[Table 1]**

| Examples | Adhesive composition (% by mass) | Base material to which adhesive is applied | Evaluation results |
|---|---|---|---|
| 3 | Commercial adhesive plaster (Nitrea tape, | (Nitrea tape is used as it is) | (1) |
| | Nitto Denko Corporation) | Total thickness: 150 µm | |
| 4 | HiPAS10 adhesive 100% | Polyethylene film | (1) |
| | | Thickness: 50 µm | |
| 5 | HiPAS10 adhesive 89% | Polyethylene film | (1) |
| | Isopropyl myristate 10% | Thickness: 50 µm | |
| | Polyacrylic acid 1% | | |
| 6 | HiPAS adhesive 87% | Urethane foam | (2) |
| | Isopropyl myristate 10% | Thickness: 50 µm | |
| | Polyacrylic acid 3% | | |
| 7 | HiPAS adhesive 50% | Polyethylene foam | (3) |
| | Isopropyl myristate 10% | Thickness: 1 mm | |
| | Polyacrylic acid 40% | | |
| 8 | HiPAS adhesive 20% | Urethane foam | (2) |
| | Isopropyl myristate 10% | Thickness: 1 mm | |
| | Polyacrylic acid 70% | | |
| 9 | HiPAS adhesive 10% | Urethane foam | (4) |
| | Isopropyl myristate 10% | Thickness: 1 mm | |
| | Polyacrylic acid 80% | | |

### Examples 10 and 11

A minute projection patch of 10 mm × 20 mm similar to that manufactured in Example 2 was bonded onto an adhesive tape cut into 16 × 26 mm for use. Over the entire minute projections, 0.2 g of "HURRICAINE GEL" (containing 200 mg of ethyl aminobenzoate in 1 g of gel ointment) was spread. The composition of the adhesive of the adhesive tape was the same as that of the adhesive used in Example 7, and a base material for an adhesive tape was a polyethylene film in Example 10 and a polyurethane foam in Example 11. Two type of minute projection patches with adhesive tapes were pressed against the human upper arm, and immediately after that, the state of projection of HURRICAINE GEL to the periphery of the adhesive tape was confirmed. In the tape configuration of Example 10, the projection of the gel ointment was observed at the edge portion of the tape, but the projection of the gel ointment was not observed around the tape of Example 11. The effect of preventing the flow of the liniment of the polyurethane foam could be confirmed.

### Example 12

A high-density polyethylene sheet having a thickness of 50 µm was placed on a metal concave mold having a stainless flat surface with a maximum height of 100 µm by a sandblasting method, heated to 150°C, and press-molded to mold a minute unevenness sheet. The sheet was cut into 10 mm × 20 mm to manufacture a minute unevenness patch. The maximum height (Rz) of the unevenness was 90 µm. The minute unevenness patch was used by bonding a commercially available adhesive plaster onto a tape cut into 16 × 26 mm. Over the entire surface of the minute unevenness, 0.15 g of a dental local anesthetic "NEOZALOCAIN PASTE" (containing 250 mg of ethyl aminobenzoate and 50 mg of parabutylaminobenzoic acid diethylaminoethyl hydrochloride in 1 g of paste) was spread. In this state, the minute unevenness patch on which the drug was placed were applied to the upper jaw parts of three volunteers, and the substrate (the back surface of the minute unevenness) was pressed to bring the paste and the minute unevenness patch into close contact with the skin. After the skin application, the tape was peeled off every 5 minutes, the drug application site was lightly pressed with a toothpick, and the presence or absence of pain was self-evaluated. In all the three volunteers, no pain was felt.

### DESCRIPTION OF REFERENCE SYMBOLS

- 1: Minute projection
- 2: Adhesive tape (film)
- 3: Adhesive tape (foam)

## Claims

1. A minute projection patch intended to be applied to skin or oral mucosa, comprising a substrate and minute projections stereoscopically erected on the substrate, wherein the substrate and the minute projections are made of a water-insoluble thermoplastic polymer substance having an elastic modulus of 1.5 × 10⁴ kgf/cm² or less.

2. The minute projection patch according to claim 1, wherein the minute projections have a tip portion diameter of 10 µm to 300 µm, the minute projections have a length of 50 µm to 1500 µm, and the substrate has a thickness of is 20 to 500 µm.

3. The minute projection patch according to claim 1 or 2, wherein the water-insoluble thermoplastic polymer substance is selected from the group consisting of polyolefin, soft silicon, and polyurethane.

4. The minute projection patch according to any one of claims 1 to 3, wherein the minute projection patch is lined with an adhesive tape.

5. The minute projection patch according to claim 4, wherein an adhesive constituting the adhesive tape contains a hydrophilic substance.

6. The minute projection patch according to claim 5, wherein the hydrophilic substance is at least one selected from the group consisting of polyacrylic acid, hyaluronic acid and a sodium salt thereof, carboxymethyl cellulose and a sodium salt thereof, polyvinyl alcohol, polyvinyl pyrrolidone, dextrin, sodium chondroitin sulfate, collagen, and crosslinked products thereof.

7. The minute projection patch according to claim 4, wherein an adhesive base material constituting the adhesive tape is a foam.

8. The minute projection patch according to claim 4, wherein an adhesive base material constituting the adhesive tape is a nonwoven fabric.

9. A local application system for shortening a time of action of a drug against skin or oral mucosa, comprising the minute projection patch according to any one of claims 1 to 8 and a liniment.

10. The local application system according to claim 9, wherein the drug is a local anesthetic agent or an antihistamine drug and is intended for pain relief or short-term achievement of anti-itch.

11. The local application system according to claim 10, wherein the local anesthetic agent is lidocaine or ethyl aminobenzoate.

12. The local application system according to claim 10, wherein the antihistamine drug is selected from the group consisting of diphenhydramine and a salt thereof, and chlorpheniramine and a salt thereof.
